# EUROPEAN PATENT SPECIFICATION

(11) **EP 2 332 950 B1**
(45) Date of publication and mention of the grant of the patent: **28.01.2015**
(21) Application number: 08877222.3
(22) Date of filing: 28.11.2008
(51) Int. Cl.: C07H 17/08, C07D 417/06, C12P 19/62, A61K 31/7048, A61P 35/00, C12R 1/01

(54) **EPOTHILONE GLYCOSIDE COMPOUNDS, COMPOSITIONS CONTAINING EPOTHILONE GLYCOSIDE COMPOUNDS AS ACTIVE COMPONENTS AND USES THEREOF**
EPOTHILONGLYCOSID-VERBINDUNGEN, ZUSAMMENSETZUNGEN MIT EPOTHILONGLYCOSID-VERBINDUNGEN ALS WIRKSTOFFEN UND ANWENDUNG DAVON
COMPOSÉS DE GLYCOSIDE D'ÉPOTHILONE, COMPOSITIONS LES UTILISANT COMME PRINCIPE ACTIF ET LEUR UTILISATION

(30) Priority: 06.10.2008 CN 200810157514
(43) Date of publication of application: 15.06.2011
(73) Proprietor: Shan Dong University, Jinan, Shandong 250100 (CN)
(72) Inventor: LI, Yuezhong, Shandong 250100 (CN); SHEN, Yuemao, ShanDong 250100 (CN); ZHAO, Lin, Shandong 250100 (CN); LU, Chunhua, Shandong 250100 (CN)
(74) Representative: Decamps, Alain René François
(86) International application number: PCT/CN2008/001946
(87) International publication number: WO 2010/040252

(56) References cited:
- EP-A1- 1 367 057
- WO-A1-93/10121
- WO-A2-02/080846
- CN-A- 1 237 970
- CN-A- 1 521 258
- HARDT I H ET AL: "New Natural Epothilones from Sorangium cellulosum, Strains So ce90/B2 and So ce90/D13: Isolation, Structure Elucidation, and SAR Studies", JOURNAL OF NATURAL PRODUCTS, AMERICAN CHEMICAL SOCIETY, US, vol. 64, no. 7, 1 July 2001 (2001-07-01), pages 847-856, XP002220541, ISSN: 0163-3864, DOI: 10.1021/NP000629F
- GUO-LI GONG ET AL: "Mutation and a high-throughput screening method for improving the production of Epothilones of Sorangium", JOURNAL OF INDUSTRIAL MICROBIOLOGY & BIOTECHNOLOGY ; OFFICIAL JOURNAL OF THE SOCIETY FOR INDUSTRIAL MICROBIOLOGY, SPRINGER, BERLIN, DE, vol. 34, no. 9, 24 July 2007 (2007-07-24), pages 615-623, XP019521832, ISSN: 1476-5535, DOI: 10.1007/S10295-007-0236-2

## Description

The invention relates to a compound of epothilone glycoside, a method of preparing the same, a pharmaceutical composition having the compound, and a use thereof as active ingredient for treatment of liver cancer, lung cancer, and breast cancer.

Epothilone is a macrolide compound with antitumor activity, among which epothilone A and epothilone B are common, having a formula of

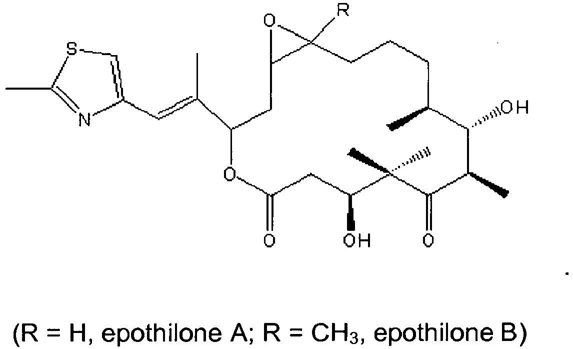

*Sorangium cellulosum*, belonging to Myxobacterales, Sorangineae, and Sorangium, is a single-cell gram-negative bacilli and widely distributed in soil. It can produce a large variety of secondary metabolites having various chemical structure and bioactive mechanism, which is similar to Streptomyces.

The main products of epothilone A and B produced in *Sorangium cellulosum* are synthesized catalytically by a multi-enzyme complex including polyketide synthase and non-ribosomal peptide synthase.

In 1993, natural epothilone having cytotoxicity was isolated by Rechenbach and Höfle. In 1995, Merch Research Laboratories verified epothilone had the similar mechanism to pacilitaxel (TAXOL^{®}). In 1996, Höfle et al. disclosed the three-dimensional chemical structure of epothilone B, in which epoxide/thiazole /ketone was involved, and thereby the compound was named as "epothilone".

Similar to pacilitaxel (TAXOL^{®}), epothilone can promote the polymerization and stability of microtubule, induce tubulin polymer to form a super-stable structure, inhibit mitosis, and thereby to prevent the proliferation of tumor cell. However, epothilone is much simpler than pacilitaxel in molecular structure, and has better water solubility and good potential for chemical modification. Additionally, epothilone exhibits high inhibitive activity on tumor cells that has strong resistance against pacilitaxel. Furthermore, due to being isolated from *Sorangium cellulosum,* epothilone can be produced by large scale fermentation.

Having the above mentioned advantages, epothilone is viewed as a good substitute of pacilitaxel and has a bright prospect for prevention and treatment of cancer.

CN1521258A relates to novel epothilone compounds and their isolation from a culture of recombinant strains of *Myxococcus xanthus* or *Sorangium cellulosum* host cells. These compounds are particularly useful for resisting tumor growth and stopping cell growth. WO93/10121A1 discloses epothilone compounds and their isolation from a fermentation culture of *Sorangium cellulosum* strain So ce90 (DSM6673), as well as their use as medicaments and as plant protecting agents. Likewise, EP1367057 discloses the synthetic preparation and purification of epothilone E and F, and their use as a therapeutic agent, in particular as a cytostatic.

Hardt et al (Journal of Natural Products, 2001, 64 (7), 847-856); CN1237970 (or EP1367057) and WO02/0870846 relate to epothilone derivatives with unmodified hydroxyl substituents at positions 3 and 7 of the macrocycle ring. These compounds were isolated from *Sporangium cellulosum* strain So ce90 or a mutated version of this strain (having an increased production of epothilone A and B over the wild type strain).

Gou-Li Gong et al. (Journal of Industrial Microbiology & Biotechnology ; 2007, 34(9), 615-623) describes the mutation of Sorangium cellulosum strain So0157-2 to improve the production of epothilones, in particular of epothilone B, which are considered as highly promising prospective anticancer agents.

Up to now, a variety of epothilone analogs has been in clinical evaluation and even on sale, including ixabepilone (aza-epothilone B BMS-247550), BMS-310705 (a water-soluble analog of epothilone B), patupilone (epothilone B, EPO906, developed by Novatis), epothilone D (KOS-862, Kosan/Sloan-Kettering /Roche), ZK-EPO, and C20-desmethyl-C20-methylsulfanyl-Epo B (ABJ879, Novatis). However there are no reports related to epothilone glycoside.

In view of the above-described problems, the invention provides an epothilone glycoside, a method of preparing the same, a pharmaceutical composition having the compound, and a use thereof as active ingredient for treatment of liver cancer, lung cancer, and breast cancer.

To achieve the above objectives, in accordance with one embodiment of the invention, there is provided an epothilone glycoside having anticancer activity, the epothilone glycoside having a formula of 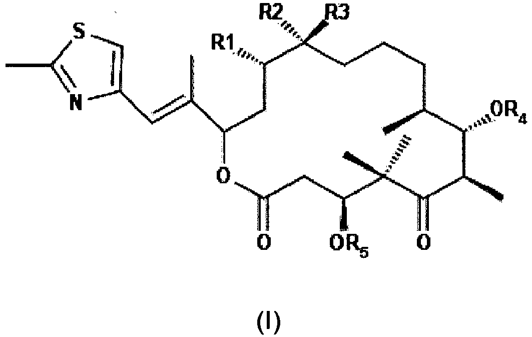 wherein

| | |
|---|---|
| R₁ + R₂ = O, R₃ = R₄ = H, R₅ = glycosyl | epothilone glycoside A-1 |
| R₁ + R₂ = O, R₃ = R₅ = H, R₄ = glycosyl | epothilone glycoside A-2 |
| R₁ + R₂ = O, R₃ = CH₃, R₄ = H, R₅ = glycosyl | epothilone glycoside B-1 |
| R₁ + R₂ = O, R₃ = CH₃, R₅ = H, R₄ = glycosyl | epothilone glycoside B-2, |

or 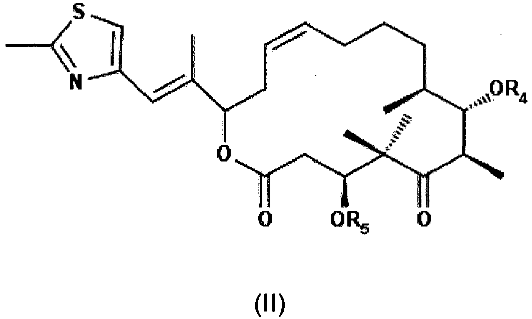 wherein

| | |
|---|---|
| R₄ = H, R₅ = glycosyl | epothilone glycoside C-1 |
| R₄ = glycosyl, R₅ = H | epothilone glycoside C-2. |

In a class of this embodiment, the epothilone glycoside is isolated from a solid or liquid fermentation product of *Sorangium cellulosum* So0157-2 CCTCC NO: M 208078, *Sorangium cellulosum* So0157-2 CCTCC NO: M 208078 being deposited in China Center for Type Culture Collection (Wuhan University, Wuhan, China) on May 27, 2008.

In accordance with another embodiment of the invention, there is provided a pharmaceutical composition comprising a therapeutically effective amount of epothilone glycoside of formula (I) or (II) and a pharmaceutically acceptable excipient for treatment and prevention of cancer.

In a class of this embodiment, the cancer is liver cancer.

In a class of this embodiment, the cancer is lung cancer.

In a class of this embodiment, the cancer is breast cancer.

In a class of this embodiment, the pharmaceutically acceptable excipient is a diluent such as water; a filler such as starch, sugar, and so on; an adhesive such as cellulose derivatives, alginate, gelatin, and polyvinylpyrrolidone; a wetting agent such as glycerol; a disintegrating agent such as agar, calcium carbonate, and sodium bicarbonate; an absorption enhancer such as quaternary ammonium compounds; a surface active agent such as hexadecanol; an adsorption carrier such as kaolin clay and bentonite; a lubricant such as talc, calcium stearate, magnesium, and polyethylene glycol. Other adjuvants such as flavor agents and sweeteners can also be added to the composition.

In another respect, the invention provides an epothilone glycoside of formula (I) or (II) for use as a medicament for treatment and prevention of liver cancer, lung cancer, and breast cancer.

Studies have shown the epothilone glycoside of the invention can treat and prevent liver cancer, lung cancer, and breast cancer. At the concentration of 10⁻⁶ M, the compound has strong inhibition on human liver cancer cell HepG2, and exhibits a certain inhibition on human lung cancer cell A-549 and breast cancer cell MDA-MB-435, which shows the active site of the compound can function as a chemical inhibitor of liver cancer, lung cancer, and breast cancer. Thus, the compound or the pharmaceutical composition comprising the compound can be used for preparation of an anticancer medication.

In a class of this embodiment, an administration mode of the compound/pharmaceutical composition is oral administration, nasal inhalation, rectal administration, or parenteral administration. For oral administration, the compound/pharmaceutical composition is a solid dosage form such as tablets, powders, granules, capsules, etc., or a liquid dosage form such as aqueous agents, oil-based suspension, syrup, and elixir agents. For parenteral administration, the compound/pharmaceutical composition is a solution for injection, an aqueous agent, or an oil-based suspension. Preferably, the dosage form is tablets, coated tablets, capsules, suppositories, nasal sprays and injections, and more preferably, is a formulation released at a specific site of intestine.

In a class of this embodiment, the dosage form is produced by conventional methods of pharmaceutical field, for example, mixing the epothilone glycoside with one or more excipients, and then preparing as needed.

In a class of this embodiment, the epothilone glycoside accounts for between 0.1 and 99.5 wt.% of the pharmaceutical composition, particularly between 0.5 and 95 wt.% of the pharmaceutical composition.

The effective amount of the compound is determined by administration mode, age and body weight of a patient, the type and severity of illness.

In a class of this embodiment, a daily dose of the epothilone glycoside is between 0.01 and 10 mg/Kg body weight, particularly between 0.1 and 5 mg/Kg body weight.

In a class of this embodiment, the epothilone glycoside can be administered once or several times.

For further illustrating the invention, experiments detailing an epothilone glycoside and a method of preparing the same are described below. It should be noted that the following examples are intended to describe and not to limit the invention.

### Example 1

1. Isolation of strain, deposition, isolation and purification of epothilone glycoside, and antitumor activity test (take epothilone glycoside A-1 as an example)

The soil for screening bacteria was collected from the shore of Chenghai Lake, Yunan province. The screening method was as follows.

Sterile filter paper was placed on a CNST plate medium having 25 µg/mL sterilized cycloheximide. The medium had the pH value of 7.2 and was cultured at 30°C. 2 days later, the growth of myxobacteria was observed daily under anatomical lens, and newly-born myxobacteria were transferred to a CNST fresh medium having the pH value of 10.0 for culture and purification. The culture temperature was 30°C. 5 days later, a fruiting body was observed and transferred to a sterilized *E. coli* trace in a WCX plate medium having 250 µg/mL kanamycin sulfate so as to remove a large variety of bacteria. Finally, *Sorangium cellulosum* at edge of the community was transferred to filter paper of another CNST plate medium (pH 10.0), and thereby the purification was achieved. Purified and mature fruiting body of myxobacteria on the plate was collected, transferred to sterile filter paper having a size of 1.5 x 3 cm, and preserved in a sterile tube at dry state. Based on the method, *Sorangium cellulosum* So0157 which was alkali resistant and could produce epothilone was obtained. The bacteria can be used as a starting strain for further selection.

As a starting strain, *Sorangium cellulosum* So0157 was further acclimation-induced by repeated solid-liquid continuous interval culture.

Specifically, *Sorangium cellulosum* So0157 was cultured invertedly in a CNST solid medium (pH value 9.0) at 30°C. 7 days later, a fruiting body was observed and fresh cells at the edge of the community were transferred to 100 mL of liquid medium of VY/2. The cells were shake cultured for 5 days at 30°C and 200 rpm. 10 mL of the first round of fermentation broth was transferred to 90 mL of liquid medium of VY/2, and shake cultured for 5 days at 30°C and 200 rpm. Subsequently, 10 mL of the second round of fermentation broth was transferred to 90 mL of liquid medium of VY/2, and shake cultured for 5 days at 30°C and 200 rpm. Here ended the first process of acclimation-induction. 1 mL of the last round of fermentation broth was cultured invertedly in another CNST solid medium (pH value 9.0) at 30°C to initiate the next culture process. After several processes, the obtained strain was shake cultured, and by evaluating the growth state and determining the yield of epothilone, a high-yield strain of epothilone was obtained. The strain was identified as myxobacteria by the State Key Laboratory for Microbial Technology, Shandong University, and 16S rDNA sequence information thereof had been published (DQ256394.1). The strain was deposited in China Center for Type Culture Collection (Wuhan University, Wuhan, China) on May 27, 2008, under deposition information: *Sorangium cellulosum* So0157-2, CCTCC NO: M 208078.

A formula of the CNST medium was: KNO₃ 0.5 g/L, Na₂HPO₄ 0.25 g/L, MgSO_{4·}7H₂O 1 g/L, FeCl₃ 0.001 %, trace element solution 1 ml/L, agar 1.5%, and the pH value was adjustable as needed. The formula was sterilized, a plate medium was prepared, and sterile filter paper was placed after cooling solidification.

A formula of the WCX medium for purifying the strain was (by weight percent): CaCl₂·2H₂O 0.15%, agar 1.6%, the pH value was adjusted with KOH to 7.0. After sterilization, 25 µg/mL sterilized cycloheximide was added. After forming a plate medium, living *E*. *Coli* was inoculated on the surface by densely crossing so as to induce the formation of a fruiting body of myxobacteria. *E. coli* was cultured with conventional LB culture medium.

Aformula of the VY/2 medium for acclimation-inducing the strain was (by weight percent): active yeast, 0.5%; CaCl₂ 0.08%; VB₁₂ 0.5 µg/mL; with pH 9.0.

A formula of the trace element solution was MnCl_{2·}4H₂O 0.1 g/L, CoCl₂ 0.02 g/L, CuSO₄ 0.01 g/L, Na₂MoO₄·2H₂O 0.01 g/L, ZnCl₂ 0.02 g/L, LiCl 0.005 g/L, SnCl₂·2H₂O 0.005 g/L, H₃BO₃ 0.01 g/L, KBr 0.02 g/L, and Kl 0.02 g/L.

Studies showed: *Sorangium cellulosum* So0157-2, CCTCC NO: M 208078 produced epothilone compound. By methods of solid fermentation, LC-MS, and activity tracking, not only epothilone A/B/C was detected, but also epothilone glycoside A-1, A-2, B-1, B-2, C-1, and C-2 having antitumor activity were obtained.

The molecular ion peak of epothilone glycoside A-1, A-2, B-1, B-2, C-1, and C-2 which were glycosides of epothilone A/B/C was also detected with LC-MS.

1.1 The fermentation of *Sorangium cellulosum* So0157-2, CCTCC NO: M 208078 and the extraction of a compound

*Sorangium cellulosum* So0157-2, CCTCC NO: M 208078 was transferred from a solid medium CNST to a solid plate M26 and cultured by conventional method at 30°C. 3-4 days later, the bacteria were collected, transferred to 50 mL of liquid medium M26, and shake cultured at 30°C. 4-5 days later, the growth of the bacteria entered logarithmic growth phase. Subsequently, the bacteria was dispersed by a spinner bottle and transferred to another liquid medium M26 for amplification. Used as seeds for solid fermentation, the amplified bacteria were centrifugated, washed with sterile water, smeared on filter paper placed in a culture medium CNST, and cultured at 30°C. 3-4 days later, the growth of the bacteria entered logarithmic growth phase. Based on a percent of 2%, a layer of resin XAD16 was coated on the filter paper. After 7-9 days' culture at 30°C, the strain entered the secondary metabolism phase completely.

A formula of the medium CNST was: KNO₃ 0.5 g/L, Na₂HPO₄ 0.25 g/L, MgSO₄·7H₂O 1 g/L, FeCl₃ 0.001 %, trace element solution 1 ml/L, agar 1.5%, the pH value 7.2. The formula was sterilized, a plate medium was prepared, and sterile filter paper was placed after cooling solidification.

formula of the medium M26 was: potato starch 8 g/L, yeast extract powder 2 g/ L, peptone 2 g/L, glucose 2 g/L, MgSO₄·7H₂O 1 g/L, CaCl₂ 1 g/L, EDTA-FeCl₃ 1 ml/L, trace element solution 1 ml/L, the pH value 7.2. Upon preparation of a solid medium, 12 g/L agar powder was needed.

A formula of the trace element solution was MnCl₂·4H₂O 0.1 g/L, CoCl₂ 0.02 g/L, CuSO₄ 0.01 g/L, Na₂MoO₄·2H₂O 0.01 g/L, ZnCl₂ 0.02 g/L, LiCl 0.005 g/L, SnCl₂·2H₂O 0.005 g/L, H₃BO₃ 0.01 g/L, KBr 0.02 g/L, and Kl 0.02 g/L.

After 7-9 days' culture, the solid plate was collected, *Sorangium cellulosum* So0157-2 and the resin XAD were scraped by a sterile shovel, and the filter paper degraded by *Sorangium cellulosum* was collected by a tweezer. The collected samples were placed in an oven (40°C) for removal of excess water and immersed with methanol. The resultant immersion solution was filtered with filter paper and dried at 40°C to yield an extract comprising epothilone glycoside A-1, A-2, B-1, B-2, C-1, and C-2.

10 L of fermented *Sorangium cellulosum* So0157-2, CCTCC NO: M 208078 and the resin XAD were immersed with methanol. The resultant immersion solution was filtered with filter paper and dried at 40°C to yield 1.25 g of extract comprising epothilone glycoside A-1, A-2, B-1, B-2, C-1, and C-2.

### 1.2 The isolation and purification of epothilone glycoside A-1

The fermentation extract of *Sorangium cellulosum* So0157-2, CCTCC NO: M 208078 was isolated by medium-pressure liquid chromatography (RP-18, 80 g), eluted with methanol-water system, i.e., 50% 1500 mL to yield M1+M2 (940 mg), 65% 700 mL (M3, 100 mg), 75% 700 mL (M4, 250 mg), and eluted with 300 mL of methanol (M5, 82 mg). The eluates were measured by TLC and developed with petroleum ether-acetone (3 : 2). The eluates M3 and M4 had spots that could be colored by an alkaloid reagent, and the spot from the M3 had large polarity.

The eluate M3 was isolated with gel column chromatography and eluted with methanol. The resultant eluates were collected automatically with each tube about 3 mL (2,600 seconds), measured by TLC, and developed with chloroform-methanol (10 : 1). The eluates 17-22 (44 mg) and 23-24 (11 mg) were combined respectively. The eluates 17-22 was further isolated with gel column chromatography and eluted with methanol. The resultant eluates were collected automatically with each tube about 3 mL (2,600 seconds), measured by TLC, and the eluates 5-8 (35 mg) were combined. The combined eluate was isolated with normal phase column chromatography, i.e., the chromatographic column was saturated with 0.8 g of silicone petroleum ether, and the sample was dissolved with chloroform, loaded, eluted with petroleum ether-ethyl acetate (10 : 1, 41 mL; 5 : 1, 48 mL) and chloroform-methanol (30 : 1) separately to yield a main component (25 mg). The component was further isolated with normal phase column chromatography, i.e., the chromatographic column was saturated with 0.6 g of silicone petroleum ether, and the sample was dissolved with chloroform, loaded, eluted gradiently with chloroform-methanol (40 : 1, 41 mL; 35 : 1, 36 mL; 30 : 1, 31 mL). 3-4 mL/tube of eluates were collected, measured by TLC, and the eluates 4-6 (13 mg) and 7-10 (5 mg) were combined respectively. The combined eluate 7-10 was measured by TLC and developed with a variety of developers to yield a single spot, which showed a pure compound was obtained. The compound was designated a code with EPO-E (NMR spectroscopy was measured with CDCl₃ as solvent).

### 2. The structural identification of the compound EPO-E

ESI-MS showed the quasi-molecular ion peak of the compound EPO-E was m/z 626.4 [M + H]⁺ and 648.3 [M + Na]⁺, so the molecular weight of the compound was 625. The compound had a fragment peak m/z 492 after m/z 133 split off. High resolution fast atom bombardment mass spectrometry showed the formula of the compound was C₃₁H₄₇NO₁₀S (HRFAB-MS, measured value: m/z 625.7706, calculated value: m/z 625.2921).

C-NMR (comprising DEPT) of the compound EPO-E had 31 signals, comprising 6 methyl, 7 methylene, 12 methine, and 6 quaternary carbon. Based on the signals of ¹H NMR spectrum at δ 5.21 (s, H-1', the anomeric proton), 3.91 (s, H-2'), 4.00 (m, H-3'), 4.31 (m, H-4'), 3.85 (H-5'), and 3.78 (dd, H-5'), the signals of ¹³C NMR spectrum at δ □108.7 (C-1'), □79.0 (C-2'), 78.2 (C-3'), 88.1 (C-4'), and 66.2 (C-5'), the signals of HMQC spectrum, and the signals of HMBC spectrum, a unit of α-D-ribofuranosyl was determined. Further studies on the signals of HMQC spectrum and HMBC spectrum showed the compound was epothilone A (corresponding data were listed in Table 1). Based on the long-range correlation between the C-1' proton and C-3, it was determined that C-3 of epothilone A was substituted with a glycoside. Thus, the compound EPO-E was epothilone glycoside, a novel compound, named as epothilone glycoside A-1.

**Table 1 NMR data of the compound EPO-E**

| **No.** | **¹H*^{b}*** | **¹³C** | **HMBC** |
|---|---|---|---|
| 1 | | 170.2s | |
| 2 | 2.59 (m) | 38.5t | C-1, C-3, C-4 |
| 3 | 4.21 (dd, 3.2, 9.4) | 79.1d | |
| 4 | | 52.9s | |
| 5 | | 219.5s | |
| 6 | 3.15 (dq, 4.7, 6.8) | 43.8d | C-25, C-8, C-5 |
| 7 | 3.78 (t, 4.6) | 74.5d | C-25, C-28, C-9, C-8, C-6, C-5 |
| 8 | 1.70 (m, 2H) | 36.2t | |
| 9 | 1.45 (m, 2H) | 31.9t | |
| 10 | 1.62 (m, 2H) | 23.3t | C-12 |
| 11 | 1.86 (m), 1.46 (m) | 27.4t | |
| 12 | 2.95 (dt, 3.2, 9.1) | 57.4d | |
| 13 | 3.07 (dt, 3.5, 9.4) | 54.8d | C-12 |
| 14 | 2.14 (m), 1.94 (m) | 32.0t | C-14, C-27, C13, C-15 |
| 15 | 5.48 (dd, 1.7, 8.6) | 77.9d | C-27, C-14, C-13, C-17, C-1 |
| 17 | | 137.5s | |
| 18 | 6.61 (s) | 117.0d | C-17, C-15, C-27 |
| 19 | | 151.5s | |
| 20 | 7.00 (s) | 121.4d | C-21, C-19 |
| 21 | | 166.0s | |
| 22 | 2.71 (s) | 19.0q | C-21, C-19, |
| 23 | 1.15 (s) | 20.1q | C-24, C-4, C-5, C-3 |
| 24 | 1.36 (s) | 21.7q | C-23, C-4, C-5, C-3 |
| 25 | 1.18 (d, 6.8) | 14.1q | C-6, C-7 |
| 26 | 1.05 (d, 7.0) | 17.3q | C-9, C-8, C-7 |
| 27 | 2.08 (s) | 15.1q | C-15 |
| 1' | 5.21 (s) | 108.7d | C-3, C-4' |
| 2' | 3.91 (br s) | 79.0d | C-3', C-4' |
| 3' | 3.98 (br s) | 78.2d | C-4' |
| 4' | 4.31 (q, 2.2) | 88.1d | C-2' |
| 5' | 3.87 (dd, 2.7, 12.0) | 62.2t | C-2', C-3' |
| | 3.79 (dd, 2.2, 12.0) | | |

The data of ¹H, ¹³C NMR, and HMBC were measured at room temperature, with CDCl₃ as solvent, and at 600 MHz, 150 MHz, and 600 MHz respectively.

In Table 1, without extra explanation, the proton signals (¹H^{b}) were normalized as ¹H.

### 3. Experiments of antitumor activity of epothilone glycoside A-1

Screening method: methyl-thiazol-tetozolium (MTT) reduction method and sulforhodamine B (SRB) protein staining method.

Cell strains: human liver cancer cell HepG2, human lung cancer cell A-549, and breast cancer cell MDA-MB-435.
Reaction time: 48-72 hrs

### Results:

No effect: 10⁻⁵ mol/L < 85%
Weak effect: 10⁻⁵ mol/L ≥ 85% or 10⁻⁶ mol/L > 50%
Strong effect: 10⁻⁶ mol/L > 85% or 10⁻⁷ mol/L > 50%

The detailed results were listed in Table 2.

**Table 2 Inhibition rate of epothilone glycoside A-1 on human liver cancer cell HepG2, human lung cancer cell A-549, and breast cancer cell MDA-MB-435**

| Concentration (M) | 10⁻⁴ | 10⁻⁵ | 10⁻⁶ | 10⁻⁷ | 10⁻⁸ | IC₅₀ (µM) | 95% confidence limit |
|---|---|---|---|---|---|---|---|
| Cell strain | | | | | HepG2 | | |
| Inhibition rate | 100 | 99.9 | 93.3 | 41.5 | 15.7 | 0.07 | 0.03-0.15 |
| Cell strain | | | | | A-549 | | |
| Inhibition rate | 90.6 | 83.7 | 72.0 | 0 | 0 | 6.47 | 0.56-75.29 |
| Cell strain | | | | | MDA-MB-435 | | |
| Concentration (µM) | 1 | 2 | 4 | 8 | 10 | IC₅₀ (µM) | 95% confidence limit |
| Inhibition rate | 18.9 | 55.0 | 76.1 | 84.6 | 98.2 | 2.07 | 0.18-33.6 |

Conclusion: as shown in Table 2, at the concentration of 10⁻⁶ M, epothilone glycoside A-1 had strong inhibition on human liver cancer cell HepG2 and weak inhibition on human lung cancer cell A-549 and human breast cancer cells MDA-MB-435. Therefore, the compound can selectively inhibit the cancer cells, and the active site of the compound can function as a chemical inhibitor of cancer.

### Example 2

| Dosage form | Component | Mass |
|---|---|---|
| Tablet | Epothilone glycoside A-1 | 1 mg |
| | Lactose | 182 mg |
| | Cornstarch | 54 mg |
| | Magnesium stearate | 3 mg |

Preparation method: Epothilone glycoside A-1 was mixed with lactose and cornstarch. The resultant mixture was uniformly wet with water, screened, dried, screened again, magnesium stearate added, and tabletted. Each tablet was 240 mg with 1 mg of epothilone glycoside A-1.

### Example 3

| Dosage form | Component | Mass |
|---|---|---|
| Capsule | Epothilone glycoside A-1 | 1 mg |
| | Lactose | 197 mg |
| | Magnesium stearate | 2 mg |

Preparation method: Epothilone glycoside A-1 was mixed with lactose and magnesium stearate. The resultant mixture was screened, mixed uniformly, and packed in a hard gelatin capsule. Each capsule was 200 mg with 1 mg of epothilone glycoside A-1.

### Example 4

| Dosage form | Component | Mass |
|---|---|---|
| Ampoule | Epothilone glycoside A-1 | 1 mg |
| | NaCl | 9 mg |

Preparation method: Epothilone glycoside A-1 and NaCl were dissolved in water for injection. The resultant solution was filtered and packed in an ampoule under aseptic conditions.

### Example 5

| Dosage form | Component | Mass |
|---|---|---|
| Capsule | Epothilone glycoside B-1 | 1 mg |
| | Lactose | 197 mg |
| | Magnesium stearate | 2 mg |

Preparation method: Epothilone glycoside B-1 was mixed with lactose and magnesium stearate. The resultant mixture was screened, mixed uniformly, and packed in a hard gelatin capsule. Each capsule was 200 mg with 1 mg of epothilone glycoside B-1.

### Example 6

| Dosage form | Component | Mass |
|---|---|---|
| Ampoule | Epothilone glycoside B-1 | 1 mg |
| | NaCl | 9 mg |

Preparation method: Epothilone glycoside B-1 and NaCl were dissolved in water for injection. The resultant solution was filtered and packed in an ampoule under aseptic conditions.

### Example 7

| Dosage form | Component | Mass |
|---|---|---|
| Tablet | Epothilone glycoside C-1 | 1 mg |
| | Lactose | 182 mg |
| | Cornstarch | 54 mg |
| | Magnesium stearate | 3 mg |

Preparation method: Epothilone glycoside C-1 was mixed with lactose and cornstarch. The resultant mixture was uniformly wet with water, screened, dried, screened again, magnesium stearate added, and tabletted. Each tablet was 240 mg with 1 mg of epothilone glycoside C-1.

### Example 8

| Dosage form | Component | Mass |
|---|---|---|
| Capsule | Epothilone glycoside A-2 | 1 mg |
| | Lactose | 197 mg |
| | Magnesium stearate | 2 mg |

Preparation method: Epothilone glycoside A-2 was mixed with lactose and magnesium stearate. The resultant mixture was screened, mixed uniformly, and packed in a hard gelatin capsule. Each capsule was 200 mg with 1 mg of epothilone glycoside A-2.

### Example 9

| Dosage form | Component | Mass |
|---|---|---|
| Ampoule | Epothilone glycoside B-2 | 1 mg |
| | NaCl | 9 mg |

Preparation method: Epothilone glycoside B-2 and NaCl were dissolved in water for injection. The resultant solution was filtered and packed in an ampoule under aseptic conditions.

While particular embodiments of the invention have been shown and described, it will be obvious to those skilled in the art that changes and modifications may be made without departing from the invention in its broader aspects.

## Claims

1. An epothilone glycoside having anticancer activity, said epothilone glycoside having a formula of 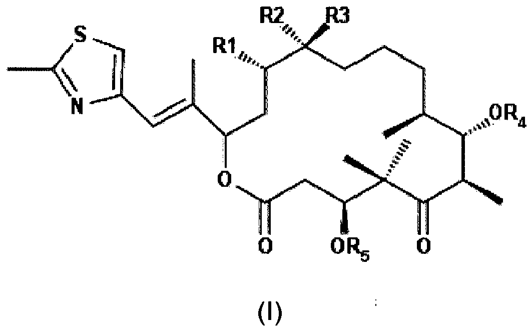 wherein
| | |
|---|---|
| R₁ + R₂ = O, R₃ = R₄ = H, R₅ = glycosyl | epothilone glycoside A-1 |
| R₁ + R₂ = O, R₃ = R₅ = H, R₄ = glycosyl | epothilone glycoside A-2 |
| R₁ + R₂ = O, R₃ = CH₃, R₄ = H, R₅ = glycosyl | epothilone glycoside B-1 |
| R₁ + R₂ = O, R₃ = CH₃, R₅ = H, R₄ = glycosyl | epothilone glycoside B-2, |
or 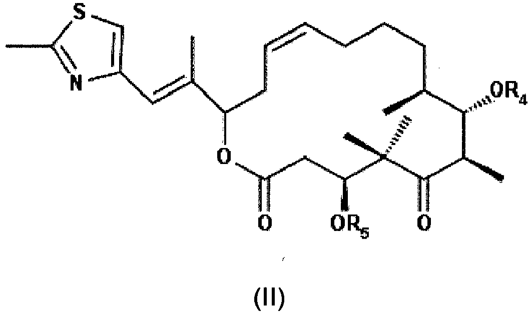 wherein
| | |
|---|---|
| R₄ = H, R₅ = glycosyl | epothilone glycoside C-1 |
| R₄ = glycosyl, R₅ = H | epothilone glycoside C-2. |

2. The epothilone glycoside of claim 1, **characterized in that** said epothilone glycoside is isolated from a solid or liquid fermentation product of *Sorangium cellulosum* So0157-2 CCTCC NO: M 208078.

3. A pharmaceutical composition comprising a therapeutically effective amount of epothilone glycoside of claim 1 and a pharmaceutically acceptable excipient for use in the treatment and prevention of liver cancer.

4. A pharmaceutical composition comprising a therapeutically effective amount of epothilone glycoside of claim 1 and a pharmaceutically acceptable excipient for use in the treatment and prevention of lung cancer.

5. A pharmaceutical composition comprising a therapeutically effective amount of epothilone glycoside of claim 1 and a pharmaceutically acceptable excipient for use in the treatment and prevention of breast cancer.

6. An epothilone glycoside of claim 1 for use as a medicament for treatment and prevention of liver cancer.

7. An epothilone glycoside of claim 1 for use as a medicament for treatment and prevention of lung cancer.

8. An epothilone glycoside of claim 1 for use as a medicament for treatment and prevention of breast cancer.

## Patentansprüche

1. Epothilonglycosid mit krebsbekämpfender Wirksamkeit, wobei das Epothilonglycosid folgende Formel aufweist 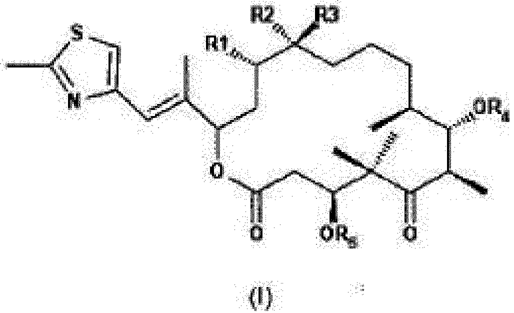 wobei
| | |
|---|---|
| R₁+R₂=O, R₃=R₄=H, R₅=Glycosyl | Epothilonglycosid A-1 |
| R₁+R₂=O, R₃=R₅=H, R₄=Glycosyl | Epothilonglycosid A-2 |
| R₁+R₂=O, R₃=CH₃, R₄=H, R₅=Glycosyl | Epothilonglycosid B-1 |
| R₁+R₂=O, R₃= CH₃, R₅=H, R₄=Glycosyl | Epothilonglycosid B-2, |
oder 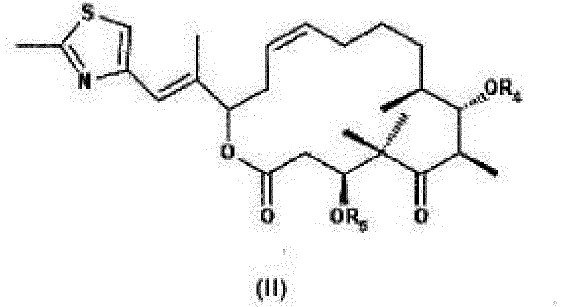 wobei
| | |
|---|---|
| R₄=H, R₅=Glycosyl | Epothilonglycosid C-1 |
| R₄=Glycosyl, R₅=H | Epothilonglycosid C-2. |

2. Epothilonglycosid nach Anspruch 1, **dadurch gekennzeichnet, dass** das Epothilonglycosid von einem festen oder flüssigen Fermentationsprodukt aus Sorangium cellulosum So0157-2 CCTCC NO: M 208078 isoliert wird.

3. Pharmazeutische Zusammensetzung, umfassend eine therapeutisch wirksame Menge von Epothilonglycosid nach Anspruch 1 und einen pharmazeutisch akzeptablen Hilfsstoff zur Verwendung bei der Behandlung und Prävention von Leberkrebs.

4. Pharmazeutische Zusammensetzung, umfassend eine therapeutisch wirksame Menge von Epothilonglycosid nach Anspruch 1 und einen pharmazeutisch akzeptablen Hilfsstoff zur Verwendung bei der Behandlung und Prävention von Lungenkrebs.

5. Pharmazeutische Zusammensetzung, umfassend eine therapeutisch wirksame Menge von Epothilonglycosid nach Anspruch 1 und einen pharmazeutisch akzeptablen Hilfsstoff zur Verwendung bei der Behandlung und Prävention von Brustkrebs.

6. Epothilonglycosid nach Anspruch 1 zur Verwendung als Medikament bei der Behandlung und Prävention von Leberkrebs.

7. Epothilonglycosid nach Anspruch 1 zur Verwendung als Medikament bei der Behandlung und Prävention von Lungenkrebs.

8. Epothilonglycosid nach Anspruch 1 zur Verwendung als Medikament bei der Behandlung und Prävention von Brustkrebs.

## Revendications

1. Épothilone glycoside ayant une activité anticancéreuse, ledit épothilone glycoside ayant la formule 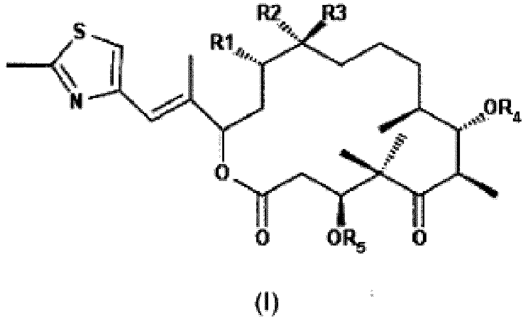 dans laquelle
| | |
|---|---|
| R₁ + R₂ = O, R₃ = R₄ = H, R₅ = glycosyle | épothilone glycoside A-1 |
| R₁ + R₂ = O, R₃ = R₅ = H, R₄ = glycosyle | épothilone glycoside A-2 |
| R₁ + R₂ = O, R₃ = CH₃, R₄ = H, R₅ = glycosyle | épothilone glycoside B-1 |
| R₁ + R₂ = O, R₃ = CH₃, R₅ = H, R₄ = glycosyle | épothilone glycoside B2-2 |
ou 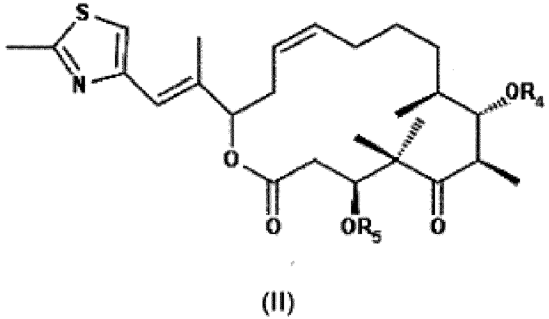 dans laquelle
| | |
|---|---|
| R₄ = H, R₅ = glycosyle | épothilone glycoside C-1 |
| R₄ = glycosyle, R₅ = H | épothilone glycoside A-2. |

2. Épothilone glycoside selon la revendication 1, **caractérisé en ce que** ledit épothilone glycoside est isolé d'un produit de fermentation solide ou liquide de Sorangium cellulosum So0157-2 CCTCC n° : M 208078.

3. Composition pharmaceutique comprenant une quantité thérapeutiquement efficace d'épothilone glycoside selon la revendication 1 et un excipient pharmaceutiquement acceptable pour son utilisation dans le traitement et la prévention du cancer du foie.

4. Composition pharmaceutique comprenant une quantité thérapeutiquement efficace d'épothilone glycoside selon la revendication 1 et un excipient pharmaceutiquement acceptable pour son utilisation dans le traitement et la prévention du cancer du poumon.

5. Composition pharmaceutique comprenant une quantité thérapeutiquement efficace d'épothilone glycoside selon la revendication 1 et un excipient pharmaceutiquement acceptable pour son utilisation dans le traitement et la prévention du cancer du sein.

6. Épothilone glycoside selon la revendication 1, pour son utilisation comme médicament pour le traitement et la prévention du cancer du foie.

7. Épothilone glycoside selon la revendication 1, pour son utilisation comme médicament pour le traitement et la prévention du cancer du poumon.

8. Épothilone glycoside selon la revendication 1, pour son utilisation comme médicament pour le traitement et la prévention du cancer du sein.
